# EUROPEAN PATENT APPLICATION

(11) **EP 0 605 742 A1**
(43) Date of publication of application: **13.07.1994**
(21) Application number: 93916319.2
(22) Date of filing: 28.06.1993
(51) Int. Cl.: A61K 31/195

(54) **AGENT WITH ADAPTOGENIC ACTIVITY**

(30) Priority: 26.06.1992 RU 5049402
(71) Applicant: KISLYAKOVA, Nonna Dmitrievna, Rostov-na-Donu, 344038 (RU)
(72) Inventor: KISLYAKOVA, Nonna Dmitrievna, Rostov-na-Donu, 344038 (RU)
(74) Representative: Lord, Hilton David
(86) International application number: RU9300138
(87) International publication number: WO9400116

(57) **Abstract**

The invention relates to medicine, in particular, to experimental ad clinical therapy an may be used for adaptogenic influence on a human organism subjected to extreme conditions or having gone through a serious illness. It is proposed to use synthetic amino acid DL-valine as an agent with adaptogenic activity. DL-valine, causing the state of nonspecifically heightened resistance of mammal's organism, in particular human organism, increases its mental and physical working capacity as well as normalizes its psychical activity. Its influence is also characterized by a significant increase of the immune protection of the organism, disappearance of some chronic inflammations, normalization of the metabolism and of the functional state of the liver, and of the cardiovascular system. The proposed agent easily synthetically obtained and functioning as a nutrition component of human food ration does not have any adverse side effect and may be widely used for prophylaxis as well as physical and psychical sanitation of the population.

## Description

The present invention relates to medicine, particularly to experimental and clinical therapy and can be utilized for producing adaptogenic effect on the organism of a person in extreme conditions or who has suffered a grave illness, and for improving his mental, physical capacity and endurance in sports exercises.

There are universally known agents of a vegetable origin exhibiting adaptogenic effect, such as extracts of ginseng and eleuterococcus roots which are capable of inducing the state of nonspecifically high resistance of the organisms of mammals.

Under experimental conditions said adaptogens created adaptation of experimental animals to unfavorable conditions.

Administration of the extract of eleuterococcus roots roots in 2 ml daily doses during two 1-month courses in autumn and winter period to workers of chemical enterprises has shown a noticeable reduction of chills in many cases.

This preparation is recommended for improving working capacity in the North. The ginseng extract is also capable of stimulating the function of cerebral cortex (see Brekhman G.I. et al "Eleuterococcus as a Means for Improving Nonspecific Resistance or Organism" AN USSR, series 5, 1965, pp. 7620764 and "New Data on Eleuterococcus and Other Adaptogens" Vladivostok, DVNTs AN USSR, 1981, pp. 79-83, 88-112). However, adaptogenic agents of vegetable origin have considerable inherent disadvantages:
1. Seasonal limitation of curative effect, i.e. most conspirous adaptogenic properties are displayed only in autumn and winter.
2. Scarcity of vegetable sources and resultant high price, e.g. of ginseng extract, which hampers their wide administration.

Among the easily available and more accessible synthetic adaptogenic agents except dibasol and, to a lesser degree, some of its derivatives at present there are no agents with a pronounced adaptogenic effect. Thus, there is a known synthetic adaptogenic agent, dibasol, taken for a prototype (see Rusin V. Ya. "On Adaptation to Cold and Heat during Muscular Training and Administration of Dibasol", J. "Pathological Physiology and Experimental Therapy", 1962, No. 6, pp. 63-65 and Rosin M.A. "State on Nonspecific High Resistance under the Effect of Some Pharmacological Agents", Ch. II in the book "Nonspecific Medicinal Prophylaxis and Therapy of Cancer" AMN USSR, L., "Medicine", 1968, pp. 27-29).

The adaptogenic properties of dibasol have been confirmed in experimental conditions by administering it subcutaneously in a physiological solution at the rate of 1 mg per 1 kg of animal body weight daily during 30 days. This stimulated adaptation of animals to cold, heat and raised their working capacity (forced swimming).

Dibasol can be used to a limited extent for prophylaxis of seasonal catarrhs of upper respiratory tracts, tracts, and anginas.

A substantial disadvantage of both dibasol and adaptogens of a vegetable origin lies in a marked seasonal limitation of their effect in autumn and winter. only.

Another serious disadvantage of dibasol is its vasodilative and hypertensive side effects so that it is generally used to arrest hypertensive crises in patients with exacerbated hypertension. (see Machkovsky M.D. "Medicinal Agents", part 1 M., "Medicine", 1985, pp. 450 - 451).

In view of the above we hereby disclose the use of a synthetic aminoacid DL-valine in the capacity of an adaptogenic agent.

Known in the prior art is the administration of DL-valine in the capacity of an aminoacid food component of aminoacid mixtures: aminofusine, steramine, trophizane used for parenteral feeding which satisfies the nitrogen needs of the organism of surgical patients after partial or complete ablation of the stomach. (see Sudzhan A.K. et al "Parenteral Feeding in Oncosurgery", M. "Medicine", 1973, pp. 70 - 71).

DL-valine is also used in experimental oncology as an antitumoral agent in vitro (see Inv. Cert. No.750787 of 28.03.1980 A 61 K 31/195 "Antitumoral Agent" by Kislyakov N.D. et al).

As distinct from the known adaptogens of vegetable origin and from the known synthetic adaptogen (dibasol). DL-valine exhibits its adaptogenic properties not only in winter and autumn but also in spring and summer inducing the state of more pronounced nonspecifically-high resistance of the organism of mammals, and man in particular, with prolonged aftereffect.

The adaptogenic effect of DL-valine on the human organism after a course of its administration is expressed in the following:
1. Substantial increase of immunoresistance of human organism to all chills (acute respiratory illness, angina, pharyngitis, post-flu complications, etc.) both in autumn-winter and spring-summer periods which is characteristic of neither analogs nor prototype of the disclosed agent.
2. Weakening and, after repeated administration of DL-valine, even vanishing of many a chronic inflammatory process, such as gastritis, cholecystitis, chronic pharyngitis, bronchitis, etc. due to development of stable resistance to harmful biological factors (bacterial organisms).
   It is worthy of note that already a few days after the beginning of DL-valine administration where apper pains at points of old and long-forgotton chronic inflammations. However, in two or three days these sensations vanish completely and do not reappear. Antiflammatory effect of other adaptogens is less expressed and little investigated.
3. Gradual normalization of metabolic processes of both general metabolism (normalization of weight) and in individual organs, e.g. cardiovascular system, liver, etc. Repeated courses deepen and consolidate this normalization.
4. Regulating and normalizing influence on the nervous activity of man, i.e. is psychic condition. Thus, in persons with more or less pronounced symptoms of astheno-depressive syndrome with a prevaling feeling of depression, uneasiness and, on the contrary, in persons susceptible to sharp excitement spells with inadequate reaction to work irritant (weakness of inhibiting proceases) their psychic state becomes normalized already within the first month of taking the DL-valine. There appear a feeling of calm, well-being and adequate reaction to various irritants. This normalization is finally consolidated by repeating the course of DL-valine after 6 - 7 months.
   Of all the known adaptogens such regulating effect on psychics of man bystimulating his cerebral cortex can be caused by ginseng alone which, however, is least available to people because of its scarcity.
   Besides, administration of ginseng in treating various degrees of asthenodepressive syndrome brings about only temporary improvement in the psychic condition state but fails to normalize it as is achieved by using the disclosed agent (see Petkov B. "On Mechanism of Action of Ginseng as a Regulator of Organism's Reactivity", in the book "New Data on Eleuterococcus and Other Adaptogens", Vladivostok, DVNTs AN USSR, 1981, pp. 106 - 112).
5. Activating effect of DL-valine on short-term and long-term memory processes which is particularly important in the gerontological aspect and in raising the mental activity. This is probably associated with the activating influence of DL-valine on metabolic processes, particularly metabolism of cholesterine and proteins in the nervous tissues of the central nervous system.
6. Substantial reduction of fatigue and stable improvement of physical activity, development of the need for physical exercises and sports training.
   Bearing in mind that this effect of DL-valine on the organism of a sportsman is fully devoid of seasonal limitations, it acquires a particular value in comparison with other adaptogens.
7. Thus, the disclosed agent has a novelty feature since at the present development state of medicine its adaptogenic function is unknown.

The invention level of the disclosed agent evidently is not based on the known achievements of modern medicine (analogs and prototypes).

The disclosed invention can be widely introduced into the practice of prophylaxis, physical and psychic improvement of people's health. The essence of the invention lies in the combination of essential features sufficient for the attainment of the result sought for.

For providing and confirming the presence of comprehensively pronounced adaptogenic properties of the new disclosed agent, DL-valine, and for revealing some mechanisms of forming these properties we have conducted a series of experiments on white nondescript rats subjected to the effect of DL-valine in comparison with control groups of the same animals (not treated with DL-valine) and with groups of animals treated with the known agent, i.e. dibasol (prototype).

The first step was to determine the optimum dose of DL-valine on milk introduced perorally (by probe into stomach) for white female and male nondescript rats.

Its has been established that the course should not exceed 3-4 weeks (once daily, five days a week). Otherwise, overdosage will reduce the adaptogenic effect of DL-valine. The daily dose of the agent depends only on the animal body weight. For example, it is 40 mg per 100 g of body weight for animals weighing 100-150 g while for those weighing about 200 g and over the dose shall be 60 mg per 100 g of animal's body weight. To prevent development of aminoacidic unbalance, the DL-valine is introduced mostly against the background of protein food ration.

Initially, the adaptogenic properties of DL-valine were checked by administering it once, 1 h before the action of the unfavourable factor, using all conventional tests (see Kovalyev G.V. et al "On Comparative Adaptogenic and Antistress Activity of Compounds with Different Chemical Structure", in the book "New Data on Eleuterococcus and Other Adaptogens", Vladivostok, DVNTs AN USSR, 1981, pp. 51 - 56) in spring and summer (April, May, June) in order to corroborate the absence of seasonal limitations in its activity.

Simultaneously, the adaptogenic activity of dibasol was tested under the same conditions for comparative assessment of adaptogenic properties of the new synthetic agent and the known medicament - dibasol (prototype).

Test 1. Assessing the influence of DL-valine on general physical activity (repeated forced swimming). Test animals were white nondescript male rats (weight 250-260 g): 10 test animals and 10 control ones. Water temperature was 21°C during both initial and repeated (three days later) swimming. Both test and control animals were swimming synchronously in pairs. Before repeated swimming the test animals were given 60 mg of DL-valine in 1 ml of milk per 100 g of body weight, 60 min before plunging into water. The medicament was administered through a probe into the stomach. The control rats received 1 ml of milk per 100 of body weight.

The time of repeated swimming of test animals was increased by 8.25±1.47 min, while that of the control animals, by 3.40±1.36 min, respectively, so that p <0.05 (statistical processing by conventional parametric method using" "Student-Fisher criterion). This points to the fact that administration of DL-valine increases physical activity of animals.

Adaptogenic effect of dibasol was checked under the same conditions on 10 test and 10 control nondescript male rats weighting 200 - 215 g. Dibasol dissolved in Ringer solution was administered to test animals subcutaneously in a does of 0.1 mg per 100 g of body weight in 0.2 ml of Ringer solution (or 1 mg/kg of body weight, a dose considered to be most effective) 60 min before repeated swimming. The control rats were also given subcutaneously 0.2 ml of Ringer solution per 100 g of body weight.

The increased time of repeated swimming in the test group was reliably lower (1.12 ± 0.37) than that in the control group (4.37 ± 1.25) at p < 0.05 which indicates that physical activity is not increased under the effect of dibasol but may even diminish, probably due to the seasonal nature of its action.

Test.II. Checking protective anticonvulsive effect of DL-valine under the damaging action of strychnine (factor of chemical nature). Test animals were white nondescript male rats (weight 200 - 250 g). The test group (7 animals) was given DL-valine in the test dose (see above) 60 min before intra-abdominal injection of toxic dose of strychnine at the rate of 0.2 mg per 100 g, i.e. 0.2 ml of 0.1%-solution per 100 g of body weight (conventional dose). The control group (7 animals) received milk.

Within the first hour 2 rats perished in the test group and seven animals in the control group. Result: 5 from 7 animals survived in the test group and none in the control group. According to the table in "Accurate Method by Fisher (from the book by V.S. Genes "Tables of Reliable Differences between Observed Groups by Qualitative Indices", "Medicine", 1964, pp. 11- 15) the differences in the survival rate after administration of DL-valine as compared with the control group are reliable (p < 0,025).

Repetition of this test under the same conditions but with subcutaneous administration of dibasol 60 min before injection of toxic dose of strychnine (same dose - 0.2 ml of solution containing 0.1 mg per 100 g of body weight) had demonstrated that 5 animals died out of 7 within the first hour and 2 survived. Out of 6 control animals 6 died and none survived (according to Fisher method p > 0,025, the difference is unreliable).

Thus, as distinct from dibasol, administration of DL-valine has exerted a pronounced protective effect against the damaging factor of chemical nature.

Test III. Checking resistance to the damaging factor of biological nature, i.e. intra-abdominal injection of ascitic cancer cells ( O 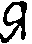 STRAIN + cancer of rats ovaries). Test animals were white nondescript female rats (weight 260 - 300 g) which were given the above-stated dose of DL-valine in the test group (8 animals) while the control group (7 animals) received milk. 60 minutes later the animals in both groups were reinoculated intra-abdominally with ascitic cells of rats' cancer of ovaries (O 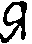 ) in the amount of 11.5 x 10⁶ cells per 100 g of body weight. Survi- val rate in days in the test group was 15 (11 - 20 days) while in the control group it was 12.1 (7 - 15 days) p = 0.05 (reliability of difference at p = 0.05), reliability of difference at p ≦ 0.05; nonparametric method of statistical processing by criterion " " of Wilkokson-Mann-Vitney (see E.K. Gubler at al in the book "Using Nonparametric Statistics Criteria for Assessing Difference between Two Observation Groups in Medico-Biological Studies". M. 1969, p. 9).

Administration of DL-valine has increased resistance of the animal organism to tumoral intoxication.

Subcutaneous introduction of dibasol under the same conditions gave survival rate of 13.4 (8 - 17 days) in the test group (7 animals) and 12.0 (7 - 17 days) in the control group, p > 0.05, i.e. with unreliable difference. Dibasol failed to improve resitance of animals to tumoral intoxication.

Test IV. Checking the antistress effect. According to the stressing method, the test was made on female rats of Vistar line (weight 80 - 100 g). 60 min before suspension the rats of stress-test group (7 animals) were given a test dose of DL-valine through a probe into the stomach while the stress-control group (7 animals) received milk. The group of intact rats (11 animals) received nothing before killing. These animals, like the first two groups (stress-test and stress-control ones) were starving for 18 h in advance. When assessing the weight changes of adrenal glands, thymus gland, spleen and the number of ulcerations of the mucous membranes in the control group (in comparison with intact animals) the result was 9 points while in the test group it was 4 points. The difference was 5 points which demonstrates a high antistress activity of DL-valine.

The result of this dibasol activity test under the same conditions (7 test animals and 7 control ones) was 7 points in the control group and 6 points in the test group. The difference was 1 point which indicates that in this case there is no antistress effect according to the assessment adopted in the method.

The obtained data are an evidence of a pronounced adaptogenic effect of DL-valine administered in a single dose regardless of the season.

The next step was to compare the prolonged effect of DL-valine (4 weeks) and of the prototype (dibasol) under the same conditions in spring and autumn, obtained from the same four tests which were described for a single administration. The produced results were identical.

The prolonged effect of DL-valine (4 weeks) was additionally checked in spring by two more tests in comparison with a control group of animals for whom DL-valine was replaced by its solvent, i.e. milk.

Test V. Checking resistance to damaging factor of physical nature, i.e. cold.

The nondescript male rats (weight 100 - 130 g) were put to a cold test in spring after daily administration (24 days running) of 40 mg of DL-valine per 100 g of body weight (test group) and milk (control group). The rats were placed for 5 min into a freezer at a temperature of minus 10 C and the result was assessed by changes in the rectal temperature of each of the test and control animals. The temperature was measured before and immediately after the cold test.

The average temperature, C, in the control group (20 animals) before the cold test was M_{an.av.} = 38.9°C while after the test it was M_{an.av.} = 38.5°C (±m = 0.115; p < 0.05) thus showing a reliable reduction of rectal temperature.

In the test group (20 animals) the respective parameters were M_{an.av.} = 39°C before and M_{an.av.} =38.9°C after the test (± m = 0.154; p 0.1). It means that there is no reduction of rectal temperature which proves a higher resistance of the organism of a mammal to considerable cooling after long-term medication with DL-valine.

Test VI. Checking radioprotecting effect of DL-valine in spring.

The first of the two tests was made on ten control nondescript female rats (100 - 200 g) which received milk in the course of four weeks, and on nine test animals treated daily within the same time period with 40 mg of DL-valine dissolved in milk per 100 g of body weight.

In the second test nine control and nine test male rats of Vistar species (130 - 200 g) were given 60 mg DL-valine per 100 g of body weight.

Upon completion of 4-week administration of DL-valine, the control and test animals were irradiated all over once on a static gamma-therapeutic apparatus AGAT-S (power rating P-245 rad/min, irradiation time 2.86 min, D = 7.0 Gr (equal to 730 r Co). Inasmuch as LD₁₀₀ for rats is 750 g for a single total irradiation with γ -rays Co⁶⁰ at an irradiation rate of 366-417 rad/min, the kind of irradiation used in the test was sublethal. The radioprotecting effect of the disclosed agent (DL-valine) was estimated by the duration of survival (in days) of the test animals in comparison with the control ones.

Experiment 1. Ten control animals lived on an average 11.1 days (8 - 20) and all perished.

Nine test animals lived M 20.2 days (9-30) and four of them survived (for statistical processing the life span was arbitrarily indicated as 30 days) p < 0.01 difference in survival days is reliable).

Experiment II. Nine control animals lived M = 12.4 (8-30) days and one survived; nine test animals lived 16.3 (9-30) days and four of them survived; p < 0.05 (difference reliable).

Hence, long-term administration of DL-valine to animals causes radioprotective effect which is another proof of high adaptogenic effect of DL-valine on the organism of mammals.

The mechanisms of forming adaptogenic effect on the organism of mammals by long-term administration of DL-valine was revealed by a series of experimental studies. The obtained data have confirmed that long-term daily administration of DL-valine for four weeks produced the following effects.
1. Favourable stimulating influence on hematogenic function of the organism of mammals. Thus, the level of hemoglobin was increased (within the upper normal limits) from M_{an.av.} = 86.7 (80 - 98) in control animals to M_{an.av.} = 96.8(86 -102) in test animals (p < 0.05), the number of erythrocytes increased from M =4420000 (3360000 - 4920000) in control animals to M = 4775000 (4560000 - 5050000) in test animals (p < 0.05), the number of reticulocytes (in per cent) increased to 2.8 (0.6 - 4.5) as compared with control animals - 1.6 (1.3 - 2.1) p < 0.05. This condition of red blood is indicative of stimulated medullary hematogenesis which, however, is devoid by any toxic effect because the number of thrombocytes stays unchanged. The condition of white blood is also changed: the number of segmental nuclear neutrophilic leucocytes diminishes and that of lymphocyctes increases within normal limits. The increased number of lymphocytes points to intensification of non-specific immune reations in the organism of animals mainly due to an increase in the number of macrolymphocytes, because this increase is accompanied by an increase se in the number of γ -globulins produced by them in the blood serum of these animals.
2. Favorable influence on protein metabolism in the organism of mammals, i.e. a considerable anabolic effect which is evidenced by an increased amount of general protein in the blood serum (to the upper normal limits) from M_{an.av.} = 7.5% (7-8.4) in control animals. to M_{an.av.} = 8.5 (8 - 94) at p < 0.01 in test animals.
   Increased absolute number of -globulins and, particularly, of -globulins: from M_{an.av.} = 0.8 (0.56 - 1.08) to M_{an.av.} = 1.6 (0.26 - 2.9) at p < 0.01 and from M_{an.av.} = 0.7 (0.44 - 0.85) to M_{an.av.} = 1.9 (0.77 - 3.48) at p < 0.01, respectively, which is associated with activization of reticuloendothelium intensifying the nonspecific immune reactions of the organism, i.e. its resistance to various unfavourable effects. The anabolic effect may also contribute to normalization of enzymic processes in such vital organs as the liver, kidneys, the cardiac muscles, etc. which are most responsible for protein metabolism in the organism of mammals.
3. Favourable influence directly on the immune system of mammals: while determining the blast-transformation reation of peripheric blood in test and control animals we have observed spontaneous (without mitogenic stimulen ants) stimulation in vivo of development of lymphoblasts from lymphocytes in peripheric blood of test animals. Thus, after the monthly administration of milk, the summary percentage of lymphoblast cells in the control group of animals amounted only to 0.30 ±0.14 while in the test group after administration of DL-valine it was 4.61 ± 1.13 at p = 0.002 (difference reliable).
   Such stimulation ensures a high functional activity of cellular immunity. The same is proved by histological data. As compared with the control animals, the lympth nodes and the spleen of test rats have undergone some changes which testify to a pronounced immune reaction of cellular type effected mainly by T-lymphocytes and to activization of immune reaction of humoral type.
4. Activization of the pituitary body-adrenal gland - thyroid gland system which ensures non-specifically high resistance of the organism of mammals.
5. Activating effect on cholesterol metabolism which is quite important for prevention of sclerotic changes in the cardiovascular system.
6. As has been established by biological tests of healthy animals the disclosed perorally administered agent - DL-valine - is completely devoid of toxic properties because its lethal dose cannot be determined even after administration on of its maximum possible quantity.

### REALIZATION OF THE INVENTION

Bearing in mind the impossibility of determining the lethal dose of DL-valine administered perorally to experimental animals and the fact of using it as a component of aminoacid mixtures for parenteral feeding of oncosurgical patients in the amount of 5 to 11 g daily per 50 kg of body weight which undoubtedly proves its full harmlessness and nutrient value (see Sudzhan A.K. in the book "Parenteral Feeding in Oncosurgery", M. "Medicine", 1973, pp. 70 - 71) we deemed it possible to check its adaptogenic effect on man, i.e. on volunteers, practically healthy persons but suffering from frequent chills, severe physical and mental fatiguability and showing symptoms of neurotic disorders.

Synthetic aminoacid - DL-valine - featuring a strong adaptogenic effect on the organism of mammals, as has been established and described above, was used for man in an empirically chosen daily dose of 0.250 to 1.500 g depending on age bracket, weight, weakening or sensitivity of the organism to medicaments and inclination to hypertension.

The disclosed invention has been used on volunteers as follows. An optimum daily dose for children after nine years of age and for old people (after 70 years) is, approximately not over 200 - 250 mg (0.2 - - 0.25 g) of DL-valine.

The daily dose for adult women is from 0.500 g to 1.000 g and 1.000 to 1.500 g for adult men. In persons suffering from hypertension a certain overdose causes a temporary rise of arterial pressure. If so, the dose shall immediately be cut in half.

The course of treatment with the disclosed agent is 2 months of which 1.5 months (6 weeks) a full daily dose is given daily, on the 7th week - half a dose daily and the last (8th week), half a dose every other day, thus gradually reducing the daily dose.

It is good practice for prophylactic purposes to repeat the course of treatment with DL-valine during especially active restructuring of the metabolic processes in the human organism caused by adaptation to cold and heat, also after a severe illness.

It is recommended that the disclosed agent be administered perorally, first dissolving the entire daily dose in half a glass of warm milk, once daily in the morning on an empty stomach (30 - 60 min before breakfast) or in the evening (before bedtime). In case of frequent insomnia spells it is preferable to take the medicament in the morning. During the treatment course the everyday ration should include protein food of animal origin such as meat, poultry, fish, dairy products, eggs, etc. once or twice a day.

The disclosed invention is illustrated by the following examples of using the new agent by volunteers.
1. A woman (F.N.S.) of 55 in a postmenopausal state periodically suffering from attacks of hypertension, stenocardia, nervous depressions, post-flu complications (rhinitis), with surplus weight, quickly getting tired of mental and physical work.

The course of DL-valine was conducted in February-March 1992, daily dose 0.500 g in half a glass of warm milk taken before bedtime. Summary course dose 30 g. i.e. 0.5 g daily within 2 months (without gradual diminishing).

### RESULTS.

1. A fortnight after the beginning of the course the arterial pressure became fully normalized, remaining so for the current period (June 1992).
2. Symptoms of stenocardia in the form of sporadic pains in the cardiac region vanished completely after the first month of treatment with the new agent and do not reappear.
3. Psychic state has normalized gradually. A feeling of calm developed along with adequate reserved reaction to all the psychic irritants and stresses.
4. Post-flu complication - rhinits - develops from time to time in a very weak form.
5. The influenza after the course of treatment lasted one day only and had no complications.
6. Physical and mental activity has increased markedly.
7. Body weight started normalizing gradually without resorting to any special diet or periodical fasting.
II. A child of nine (T.S.) suffering from chronic pharingitis, a certain unbalanced psychic state (weak inhibitory processes), frequent chills (rhinitis, laryngitis), absence of appetite, asthenic constitution, easily developing feeling of fatigue after school day.

A 2-month course of treatment with the disclosed agent was conducted in March-April of 1991 with an initial daily dose (45 days) of 0.250 mg on warm milk given before bedtime. The summary course dose was 12.5 g.

### RESULTS.

1. Chills vanished to the rest of the year.
2. Normalization of inhibitory processes was developed and consolidated. The child is active emotionally steady up to the present day (June 1992).
3. Steady appetite developed. Weight increased by 2-3 kg already in the course of medication with neither gain nor loss of weight.
4. Strong after-school fatigue vanished. School year was completed and new year started successfully. A year later (spring 1992) some weakness recurred which called for repeating a course of treatment.

The new course was started in the middle of May 1992, administered in the same dosage. A month later the appetite improved considerably, the child put on some weight, mental and physical activities improved.
III. A man of 64 (K.S.V.) suffering from mental disorders of climacteric origin (weakened inhibitory processes, inadequate reaction to weak psychic irritants), poor resistance to chills (active form of tuberculosis in his youth), easy fatiguability under physical load, symptoms of age-induced predisposition to surplus weight, low blood pressure.

A course of treatment was conducted in September-October 1991 with an initial daily dose (45 days) of 1.500 g on warm milk given before bedtime, a summary course dose of 75 g.

### RESULTS.

1. Mental state started normalizing already after 2 weeks of medication with the new agent and lasted for 1.5 months but after a sharp cancellation of the prescribed medicament (during administration of initial dose) the disorders sometimes recurred. Medication was resumed for two more weeks (entire course was 2 months) with gradual reduction of the dose. The emotional state stays normal to the present day.
2. There was not a single chill within the entire autumn-winter-spring period after the course of treatment.
3. On completion of the treatment course with the new medicament the patient showed considerable physical activity, absence of rapid mental and physical fatiguability.
4. The weight was normalized, inclination to obestity disappeared and is not observed at present (7 months since).
5. Symptoms of low blood pressure disappeared. The effect of the disclosed agent - DL-valine - which possesses pronounced adaptogenic properties without seasonal limitations and complications was checked on 12 volunteers: four men, seven women (two of them beyond 70 years of age) and one child of 9.

In all cases first of all we observed prolonged normalization of mental state (up to a year), considerable improvement of the immune protection of the organism, pronounced steady improvement of physical and mental activity and vanishing of symptoms of some chronic inflammatory processes, such as anacid and hyperacid gastritis, cholecystitis, inflammations of otorhinolaryngological organs. Other results included functional normalizing of cardiovascular system, liver, general metabolism (weight), disappearance of psoriasis symptoms (1 case).

The technical result of administering the disclosed new agent noted for raising nonspecifically the resistance and activity of the organism and normalizing its psychic activity lies in that the disclosed medicament - synthetic aminoacid - DL-valine-, being an easily synthesized and available substance functioning as a nutrient component of the food ration of man without any harmful side effects is capable, unlike other known adaptogens, of exerting a strongly expressed adaptogenic effect on the organism of man, without any seasonal limitations.

This new disclosed agent can be widely utilized for prophylaxis, for physical and mental health improvement of population, for overcoming stresses and hardships caused by restructuring of our society.

A complete harmlessness of the disclosed agent has been proved by biological studies on healthy animals and using it in the capacity of a component or aminoacid mixtures for parenteral feeding of surgical patients as has been described above.

## Claims

1. Administration of synthetic aminoacid in the capacity of an adaptogenic agent.
